# EUROPEAN PATENT APPLICATION

(11) **EP 2 347 731 A1**
(43) Date of publication of application: **27.07.2011**
(21) Application number: 11166254.0
(22) Date of filing: 20.05.2005
(51) Int. Cl.: A61F 2/06

(54) **Enhanced biological fixation of grafts**

(30) Priority: 20.05.2004 US 572806 P; 20.05.2004 US 572807 P; 20.05.2004 US 572808 P
(62) Divisional of application: 05757101.0
(71) Applicant: Med Institute, Inc., West Lafayette Indiana 47906 (US)
(72) Inventor: Leewood, Alan R., Lafayette, IN 47905 (US); Bahler, Clinton D., Indianapolis, IN 47906 (US); Sun, Jichao, West Lafayette, IN 47906 (US); Fearnot, Neal E., West Lafayette, IN 47906 (US)
(74) Representative: Ellis, Katherine Elizabeth Sleigh

(57) **Abstract**

A stent graft (1) has a substantially tubular body having a proximal and a distal end, at least the proximal end (5) comprising a region intended in the use to engage a landing zone in a vessel in the body in use, the region comprising a mechanical treatment to enhance biological fixation to the landing zone. The mechanical treatment can be provision of apertures (92), provision of relatively rigid engagement portions (17), impregnation with a SIS gel or digest (72) and mounting of a cuff or collar of SIS (60) or some other biocompatible material.

## Description

This invention relates to a graft material and more particularly to a graft material with enhanced or improved potential for biological fixation on or in the human or animal body.

This invention will be generally discussed in relation to its application to endoluminally deployed grafts and stent grafts but the invention is not so limited and can also be applied to the grafts to be applied to or in the human or animal body and where biological fixation is a desired or necessary function.

Stent grafts for endoluminal deployment into body lumens of a human or animal are generally formed from a tube of a biocompatible material and a stent or stents to maintain a lumen therethrough. Such grafts are used to span a damaged portion of the lumen such as an abdominal aortic aneurysm. Generally the stent graft engages against the wall of the lumen either side of the aneurysm in a region referred to as a landing zone. It is desirable to have some fixation to the wall of the lumen in the landing zone to ensure that the stent graft will not migrate. In particular, proximal fixation in the neck region ofthe aorta of an abdominal aortic aneurysm stent-graft is a critical function with respect to long term durability of endovascular repair. This is important because pulsating blood in the aorta can provide considerable forces on a stent graft. Ineffective fixation can result in stent-graft migration which can lead to subsequent type I endoleaks and the real possibility of subsequent intervention required by the physician to repair the leaks. The present solutions to the problem of fixation mostly depend on mechanical anchoring mechanisms. Frictional forces between the stent-graft and aortic wall are created by the interference fit between the diameters of the stent-graft and aorta wall supported by the underlying stent or stents. The practice of over-sizing a device for the lumen into which it is to be placed is directly related to the intentional creation of these frictional holding forces. The second significant fixation force is related to small hooks or barbs which completely penetrate the arterial wall. In both cases, the fixation force provided is immediate and does not require long term biological interaction. A third fixation force, tissue encapsulation, occurs in some devices over a much longer time frame (up to many months or even years). Exposed stainless steel stent struts, such as the renal stent in some forms of abdominal aortic aneurysm (AAA) stent-graft device, eventually become completely encapsulated (each strut is surrounded) by tissue growth resulting in additional and significant fixation. The interference fit and to some extent encapsulation mechanism provide sealing required to exclude the aneurysm which is the purpose ofthe device. There is a further problem that through the complex physiological process of arterial disease and aneurysm growth, frictional forces can be negated by the loss of the interference fit discussed above. That is, the neck of the aorta can further dilate due to disease, normal aging or the outward force of the stent. Thus over time it is possible to lose some or all of these mechanical normal forces. In addition, the fixation provided by barbs can be under risk due to the corrosion of the solder material and local tearing of the aortic wall.

Cell ingrowth into graft materials is sought for many different medical device applications. The biocompatible material forms a barrier that cells might be able to attach to, but cannot grow into. In the case of a vascular graft, cell ingrowth is important to avoid the formation of thrombus, create a seal, and to secure the graft intra or interluminally. The most thrombo-resistant material known is a monolayer of endothelial cells. Without cell ingrowth into a graft, it is has been virtually impossible to achieve a monolayer of endothelial cells using traditional graft materials.

Also, without cellular ingrowth, fixation and sealing of the device is left to mechanical means, which could fail over time.

Currently, for large diameter grafts such as AAA grafts, the trend is to use expanded poly tetrafluoethylene (ePTFE) or Dacron fabric with minimal porosity. Tissue ingrowth has been sacrificed to ensure that type IV endoleaks (leakage due to high porosity graft material) are eliminated. Fixation and sealing are achieved through mechanical means as discussed above and thrombus is not seen as an eminent concern due to the high flow rate in large diameter grafts.
It is an object ofthis invention provides a biological fixation mechanism which does not rely on these secondary mechanical anchoring forces or to at least provide a physician with an alternative fixation mechanism.

Throughout this specification the term distal with respect to a portion of the aorta, a deployment device or a prosthesis is the end of the aorta, deployment device or prosthesis further away in the direction of blood flow away from the heart and the term proximal means the portion of the aorta, deployment device or end of the prosthesis nearer to the heart. When applied to other vessels similar terms such as caudal and cranial should be understood.

In one form therefore the invention resides in a stent graft comprising a substantially tubular body having a proximal end and a distal end, at least the proximal end comprising a region intended in use to engage a landing zone in a vessel in the body in use, the region comprising a mechanical treatment to enhance biological fixation to the landing zone.

The mechanical treatment can be selected from the group comprising provision of apertures, provision of relatively rigid, as hereinafter described, engagement portions, impregnation with a SIS gel or digest and mounting of a SIS cuff or collar.

In an alternate form the invention comprises a stent graft comprising a substantially tubular body having a proximal end and a distal end, at least the proximal end comprising a plurality of relatively rigid engagement portions extending radially outward therefrom to engage a landing zone in a vessel in the body in use.

Preferably the plurality of relatively rigid engagement portions are placed on that portion of the stent graft which in use is expected to engage against the landing zone of the vessel as discussed above.

In this specification the term relatively rigid is intended to mean that the engagement portions are made from a material which when engaged against the wall of a lumen into which the stent graft is deployed deforms the wall at the region of engagement of the engagement portion. Hence the material of the engagement portions should be more rigid than the vessel walls.

The engagement portions may be comprised of a metal, synthetic fiber or thread or a polymeric material.

The engagement portions may be in the form of loops, coils, angled portions, buttons, spikes or other protrusions or the like.

The engagement portions may be formed onto the tubular body by stitching, adhesion or threading a further material or extra portions of the same material of the graft into the material of the tubular body.

Preferably the engagement portions are resilient so that they can be compressed into a introducer device for endoluminal deployment.

Although the process by which enhanced biological fixation is not fully understood it is suggested that the process may be as follows. The engagement portions provide a matrix of radially outward protruding portions when the stent graft is deployed and a stent associated with the graft is providing radially outward pressure. These protruding portions create a localized pressure (greater than 30 mmHg-to overcome capillary pressure) against the aorta wall initiating cell necrosis. After cell death the protruding portions will continue to impinge on the arterial wall leading to endothelial wall remodeling to accommodate the protruding portions. Each protruding portion may in time become completely encapsulated by tissue growth. In effect, the protruding portions introduced on the outside of the graft serve as scaffold for tissue (adventitia) to grow around each protruding portions creating a significant fixation and sealing mechanism.

In some embodiments the process of mounting the engagement portions onto the tubular body may create "micro-dimensional" through-thickness holes or pores which will encourage cells to grow into and through the graft and achieve further biological fixation and sealing.

In a further form the invention is said to reside in a stent graft comprising a substantially tubular body of a biocompatible graft material, the tubular body having a proximal end and a distal end, the proximal end including an array of resilient wire loops extending radially outward from the tubular body.

Preferably each wire loop is formed from a stainless steel or Nitinol wire.

The each wire loop may be in the form of a semicircle, a coil, an angled portion or the like.

At least some of the loops may be cut or otherwise severed to provide spike portions. The use of spikes to intentionally irritate the vessel wall is so that faster cell response may be expected. This may be critical for old patients, considering the fact that thrombosis/calcification may occur on the vessel wall and the cells in their vessels are not so aggressive as might be expected. The number and location of the spikes can be varied.

The wire may have a diameter of from 3 to 10 microns and the loops formed on the tubular body may extend from the tubular body from 50 to 200 microns.

The wire loops may be formed onto the tubular body by stitching, adhesion or threading into the weave of biocompatible material.

The biocompatible graft material may be Dacron, expanded polytetrafluoroethylene (ePTFE), other synthetic biocompatible materials or a naturally occurring biomaterial, such as collagen. A specially derived collagen material known as an extracellular matrix (ECM), such as small intestinal submucosa (SIS) is particularly preferred. Besides SIS, examples of ECMs include pericardium, stomach submucosa, liver basement membrane, urinary bladder submucosa, tissue mucosa, and dura mater.

Hence it will be seen that by this invention there is provided a fixation mechanism that does not rely upon purely mechanical forces to prevent movement but is based on arterial tissue growth through the scaffolding created by the stitched loops or other types of engagement portion as well as direct tissue in-growth through the pores created by the stitching process.

In a further form the invention is said to reside in a graft comprising a synthetic biocompatible graft material, at least a portion ofthe graft material having a biologically active material associated therewith.

This biologically active material will promote tissue re-growth and remodeling at an accelerated pace. Hence this invention supplements the current fixation techniques by providing an enhanced biological fixation and sealing mechanism. In effect tissue growth into the synthetic graft material is promoted by the presence of the biologically active material. In the case of a stent graft this biological fixation enhancement can be localized to the proximal and distal neck regions where attachment is critical for fixation and sealing. The fixation and sealing of a graft incorporating the biological fixation enhancement of the present invention is biological and permanent and does not require a long term and less reliable mechanical fixation mechanism which are problematic at best.

Various methods and designs can be used to associate the biologically active material with the synthetic graft material. Methods can include attaching, infusing, encapsulation, etc. the biologically active material to the graft material in ways that will survive the manufacturing process and be delivered to the neck region, for instance of a stent graft, intact until the tissue in-growth and biological fixation is complete.

The graft may be in a form of a stent graft and the biologically active material can be associated with the graft material at at least a proximal region of the stent graft in a region which may be referred to as a landing zone for engagement with a vessel wall. The biologically active material can also be associated with the graft material at a distal region landing zone.

The synthetic biocompatible material may be Dacron or expanded polytetrafluoroethylene.

The biologically active material may be formed from a naturally occurring biomaterial, such as collagen, particularly a specially derived collagen material known as an extracellular matrix (ECM), such as small intestinal submucosa (SIS). Besides SIS, examples of ECMs include pericardium, stomach submucosa, liver basement membrane, urinary bladder submucosa, tissue mucosa, and dura mater.

SIS is particularly useful, and can be made in the fashion described in Badylak et al., US Patent 4,902,508; Intestinal Collagen Layer described in US Patent 5,733,337 to Carr and in 17 Nature Biotechnology 1083 (Nov. 1999); Cook et al., WIPO Publication WO 98/22158, dated 28 May 1998, which is the published application of PCT/US97/14855. In addition to xenogenic biomaterials, such as SIS, autologous tissue can be harvested as well. Additionally Elastin or Elastin-Like Polypetides (ELPs) and the like offer potential as a biologically active material. Another alternative would be to use allographs such as harvested native valve tissue. Such tissue is commercially available in a cryopreserved state.

A first method by which a biologically active material can be associated with the synthetic biocompatible graft material may be by infusing a SIS gel into the relatively porous graft. The natural porosity of a woven fabric (which can be altered by various weaving strategies) can allow the infusing into the graft of a SIS gel by applying a vacuum on one side ofthe graft and "pulling" the SIS gel into the interstices which form the natural porosity of the graft.

A second method by which a biologically active material can be associated with the synthetic biocompatible graft material may be by vacuum press a SIS sheet onto the synthetic graft material with the use of a SIS gel as an adhesive. SIS sheet is a proven product capable of providing a collagen scaffolding enhanced with natural porcine growth factors and other proteins. The use of SIS gel as a "glue" both connects the SIS sheet to the graft as well as promoting further tissue-graft in-growth. One possible manufacturing process is to apply the SIS gel and SIS sheet on to the outside of the graft and then "vacuum-pull" the graft so that SIS gel can penetrate the graft, subsequently polymerize the composite graft in the oven at 37 °C for 20 minutes and then freeze dry and /or vacuum press the composite graft.

A third method by which a biologically active material can be associated with the synthetic biocompatible graft material may be by attaching SIS sheet by use of metal or synthetic biocompatible fiber or thread.

SIS sheet can be stitched on to a synthetic graft so as to form a secure and permanent mechanical attachment. SIS gel can then be used as a "binder" by following the steps described in the second method to fill the pores created by the stitching process and further, any protruding fibers can form a mechanical scaffolding to hold the SIS gel in place. The stitching can be done using a biocompatible thread such as a suture thread or by the use of a wire thread.
At least some of the stitching with the biocompatible thread such as a suture thread or by the use of a wire thread can extend beyond the outer surface of the stent graft and provide a plurality of relatively rigid engagement portions extending radially outward therefrom to enhance fixation.

In a further form the invention is said to reside in a graft comprising a biocompatible graft material, at least a portion of the graft material having a selected porosity to allow cell in-growth and still provide an adequate short term seal and fixation.

In a further form the invention is said to reside in a stent graft comprising a tubular body of a biocompatible graft material defining a lumen therethrough and having a proximal end and a distal end, at least a portion along the length of the proximal end of the tubular body having a selected porosity to allow cell in-growth. The porosity may be provided by an array of apertures in the biocompatible graft material. The aperture may have a diameter in the range of from 20 microns to 120 microns and a spacing of from 20 to 250 microns.

The apertures may be formed in the biocompatible graft material by the use of laser drilling. Lasers provide a small beam spot, accurate drilling depth control, and minimum heat affected area. Excimer lasers are specifically noted for theirdegree of precision and minimal damageto surrounding material. Lasers have the capability to drill holes smaller than 50 microns at varying interpore distances. In an alternative form the selected porosity may be provided by modification of the weave pattern of the biocompatible fibres which make up the graft material. For instance in the case of a stent graft a region at the proximal end of the stent graft may be woven in such a way as to provide apertures between the warp or weft fibres. Such aperture may have opening dimensions in the region of from 20 microns to 100 microns.

In a further form the invention is said to reside in a method of producing a graft material adapted for biological fixation in the human or animal body comprising the step of ablating a plurality of apertures into the graft material, the apertures having a diameter in the range of from 20 microns to 120 microns and a spacing of from 20 to 250 microns.

Preferably the ablation is done with a laser.

Hence it will be seen that there is provided by this invention a method of forming a graft and a graft formed from a material that has controlled and localized porosity of a sufficient size that will allow cell in-growth while not being sufficiently porous to allow blood leaks.

In a further form the invention comprises a stent graft comprising a substantially tubular body of a biocompatible graft material, the tubular body having a proximal end and a distal end, at least the proximal end including a cuff or collar of SIS or some other biocompatible material to create a localized pressure on a landing zone in use thereby initiating cell necrosis and subsequent endothelial wall remodeling against the aorta wall to accommodate the protruding portions to enhance biological fixation of the stent graft to the landing zone.

Hence it will be seen that there is provided by this invention a stent graft that has enhanced biological fixation.

This then generally describes the invention but to assist with understanding reference will now be made to the accompanying drawings which show preferred embodiments of the invention.
In the drawings:
Figure 1 shows a perspective view of a portion of a stent graft incorporating a biological fixation arrangement according to one embodiment of the present invention;
Figure 2 shows a cross sectional view of the stent graft along the line 2 - 2' in Figure 1;
Figure 3 shows a perspective view of a portion of a stent graft incorporating the biological fixation arrangements according to an alternative embodiment ofthe present invention;
Figure 4 shows a cross sectional view of the stent graft along the line 4 - 4' in Figure 3;
Figures 5a to 5d show details of various shapes of engagement portions according to an embodiment the present invention;
Figure 6 shows a perspective view of a portion of a stent graft incorporating an alternative embodiment of a biological fixation arrangement according to the present invention;
Figure 7 shows an enlarged view of a portion of the graft material of Figure 6 showing a method by which SIS can be associated with the graft material;
Figure 8 shows an enlarged view of a portion of a graft materialshowing an arrangement by which SIS can be associated with and into the graft material;
Figure 9 shows a second stage of the process started in Figure 8;
Figure 10 shows an enlarged view of a portion ofthe resulting graft material;
Figure 11 shows a perspective view of a portion of a stent graft incorporating another embodiment of the biological fixation arrangement according to the present invention;
Figure 12 shows an enlarged view of a portion of the graft material of Figure 11 showing how SIS can be impregnated into the graft material;
Figure 13 shows an enlarged view of a portion of the resulting graft material of Figure 12;
Figure 14 shows a perspective view of a portion of a stent graft incorporating an alternative embodiment of a biological fixation arrangement according to the present invention; and
Figure 15 shows an enlarged view of a portion of the graft material shown in Figure 14.

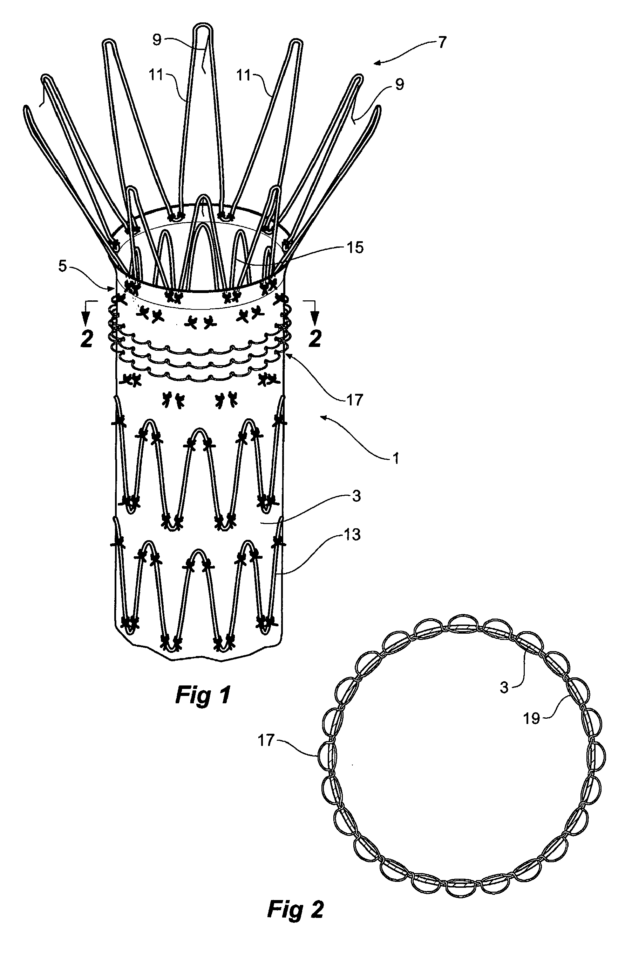

Figure 1 shows a perspective view of a portion of a stent graft incorporating biological fixation arrangements according to one embodiment of the present invention. The stent graft 1 has a tubular body 3 of a biocompatible material such as a woven Dacron. Along the length of the tubular body 3 there are self expanding Z stents 13 outside the tubular body and stitched to the tubular body. At the proximal end 5 there is an internal self expanding Z stent 15 again stitched to the body. When the stent graft 1 is deployed into a vessel of a human or animal body such as an aorta this proximal Z stent 15 provides pressure against the wall of the aorta in the landing zone and with the Z stent 15 within the tubular body an essentially smooth outer surface is presented to the wall of the aorta to provide a degree of initial sealing.

At the proximal end 5 of the stent graft 1 there is also a proximally extending uncovered self expanding Z stent 7. This proximally extending Z stent 7 has barbs 9 extending from struts 11 of the stent 7. When the stent graft 1 is deployed into a vessel of a human or animal body such as an aorta the barbs 9 engage into the wall of the aorta and provide a purely mechanical immediate fixation of the stent graft into the vessel. There is a problem with migration with pulsating blood flow, for instance, which may cause the barbs to tear the wall of the aorta. An added long term biological fixation is therefore provided according to this invention.

The added long term biological fixation is provided by a plurality of engagement portions 17 which are fastened to the tubular body towards the proximal end 5 thereof and which extend radially outwards from the tubular body. In this embodiment the plurality of engagement portions 17 are provided by loops of stiff polymer thread, stainless steel or Nitinol wire. The stiff polymer thread can for instance be a suture thread. The loops can be stitched to the tubular body by using a sewing machine or other similar machine. During the sewing process, the small loops 17 can be created on the outside of the tubular body of the stent graft 1. There can be several rows of loops as shown in Figure 1.

Figure 2 shows a cross sectional view of the stent graft along the line 2 - 2' in Figure 1. The tubular body 3 of the stent graft 1 has the wire loops 17 stitched into it with an internal thread 19 catching the ends of each loop.

Although a semicircular shape is shown in Figure 2, the loops can be stitched in many shapes, and multiple bands of loops can be stitched to the areas where the maximal fixation is desired. The loops do not necessarily have to be circular in shape, rather can take sharper forms such as an ellipse or right angled.

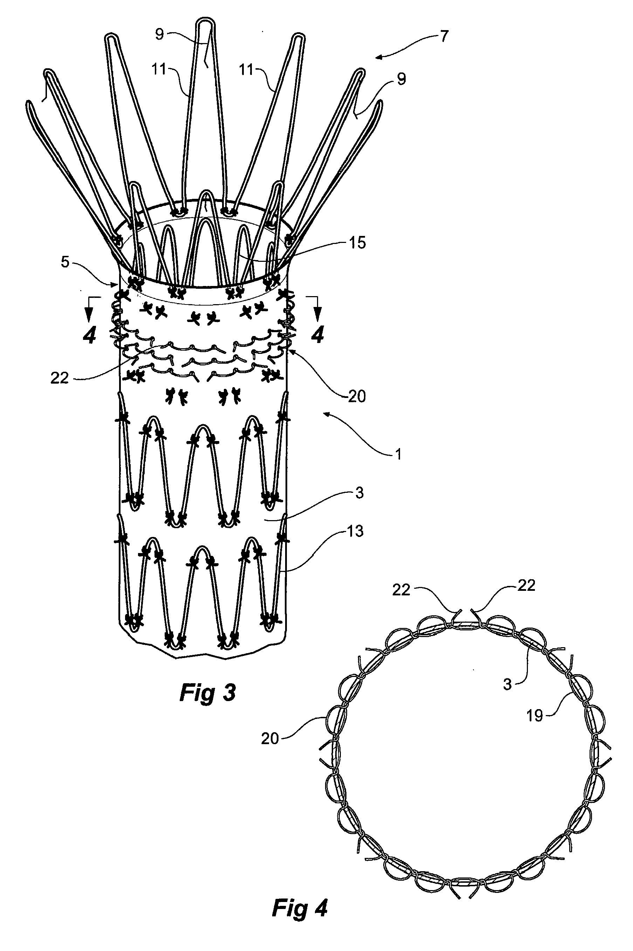

Figure 3 shows a perspective view of a portion of a stent graft incorporating the biological fixation arrangements according to an alternative embodiment ofthe present invention. In this embodiment the same reference numerals are used for corresponding components of the embodiment shown in Figure 1.

The stent graft 1 has a tubular body 3 of a biocompatible material such as a woven Dacron. Along the length of the tubular body 3 there are self expanding Z stents 13 outside the tubular body and stitched to the tubular body. At the proximal end 5 there is an internal self expanding Z stent 15 again stitched to the body. When the stent graft 1 is deployed into a vessel of a human or animal body such as an aorta this proximal Z stent 15 provides pressure against the wall of the aorta in the landing zone and with the Z stent 15 within the tubular body an essentially smooth outer surface is presented to the wall of the aorta to provide a degree of initial sealing.

At the proximal end 5 ofthe stent graft 1 there is also a proximally extending uncovered self expanding Z stent 7. This proximally extending Z stent 7 has barbs 9 extending from struts 11 of the stent 7. When the stent graft 1 is deployed into a vessel of a human or animal body such as an aorta the barbs 9 engage into the wall of the aorta and provide a purely mechanical immediate fixation of the stent graft into the vessel.

An added long term biological fixation is provided according tothis invention to provide added resistance to the force of pulsating blood.

The added long term biological fixation of this embodiment is provided by a plurality of engagement portions 20 which are fastened to the tubular body towards the proximal end 5 thereof in that portion which is intended to engage with the landing zone when deployed as discussed above and which extend radially outwards from the tubular body. In this embodiment the plurality of engagement portions 20 are provided by loops of stiff polymer thread, stainless steel or Nitinol wire. The stiff polymer thread can for instance be a suture thread. The loops can be stitched to the tubular body by using a sewing machine or other similar machine. During the sewing process, the small metal wire loops 20 can be created on the outside of the tubular body of the stent graft 1. There can be several rows of loops as shown in Figure 3. At least some of the loops 20 are cut or otherwise severed at an apex of the loop to provide a pair of spikes 22 to engage against the wall of a vessel when deployed to intentionally irritate the vessel wall is so that faster cell response may be expected. This may be critical for old patients, considering the fact that thrombosis/calcification may occur on the vessel wall and the cells in their vessels are not so aggressive as might be expected. The number and location of the spikes can be varied. In this embodiment about one in three loops have been cut but alternate or every loop could be formed in to spikes.

Figure 4 shows a cross sectional view of the stent graft along the line 4 - 4' in Figure 3. The tubular body 3 of the stent graft 1 has the wire loops 20 stitched into it with an internal wire 19 catching the ends of each loop. At least some of the loops are cut or otherwise formed into a spike or spikes at an apex of the loop to provide a pair of spikes 22 to engage against the wall of a vessel when deployed.

Although a semicircular shape for the loops is shown in Figure 4, the metal wire can be stitched in many shapes, and multiple rings of wire can be stitched to the areas where the maximal fixation is desired. The wire "loops" do not necessarily have to be circular in shape, rather can take sharper forms such as an ellipse or right angled.

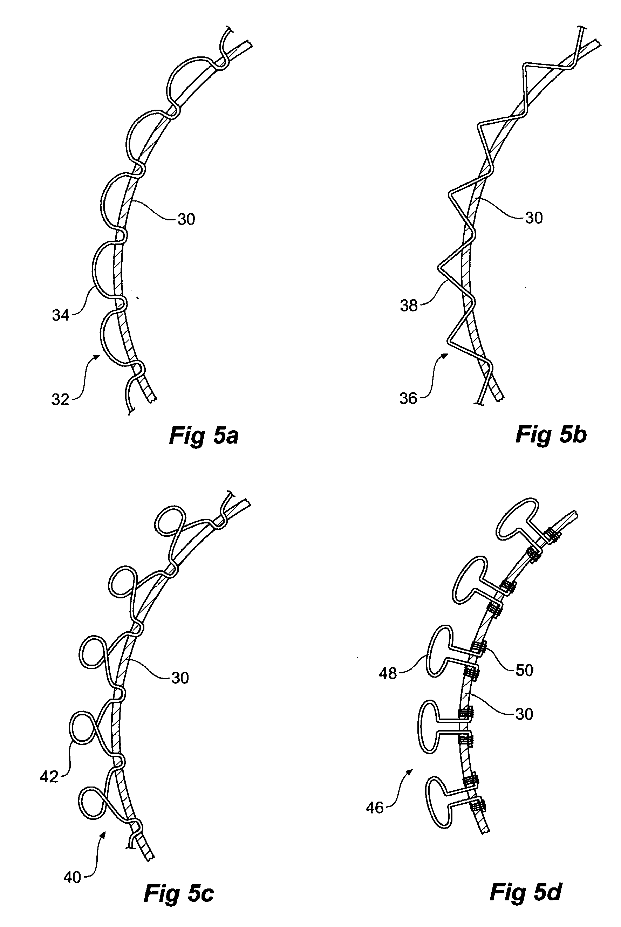

Figures 5a to 5d show details of various shapes of engagement portions according to the present invention. In each case the engagement portions extend from the tubular body 30 of the stent graft. In Figure 5a the engagement portions 32 are formed from a wire 34 which is stitched through the tubular body 30 in a series of semicircles. In Figure 5b the engagement portions 36 are formed from a wire 38 which is stitched through the tubular body 30 in a series of angled portions. In Figure 5c the engagement portions 40 are formed from a wire 42 which is stitched through the tubular body 30 in a series of coils or helices. In Figure 5d the engagement portions 46 are individually formed from wires 48 which are formed into button shapes and mounted into the tubular body 30 and stitched through the tubular body 30 by stitches 50.

As discussed earlier the material from which the engagement portions are made is selected so that it is more rigid than the vessel wall ofthe vessel into which it is placed so that it deforms the wall, causes necrosis and tissue growth around the engagement portions. Thus, a material such as Nitinol in the form of a wire may provide an advantage due to its high degree of elastic (recoverable) strain. Other materials such as plastics materials, relatively stiff suture threads such as monofilament suture thread can also be used. The materials should also be resilient so that they can be compressed during deployment and upon release they can extend out to engage the wall of the aorta or other vessel.

The engagement protrusions may have nano-scale surface features which may enhance tissue adhesion and spreading onto the surface of the engagement protrusions.

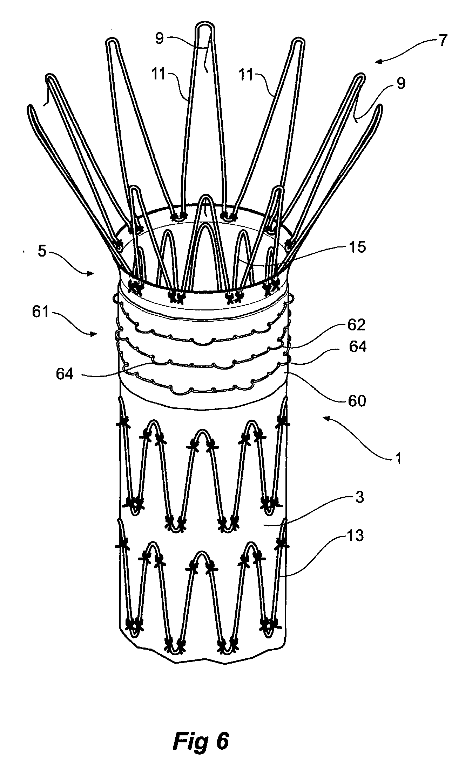

Figure 6 shows a perspective view of a portion of a stent graft incorporating the biological fixation arrangements according to an alternative embodiment ofthe present invention. In this embodiment the same reference numerals are used for corresponding components of the embodiment shown in Figure 1.

The stent graft 1 has a tubular body 3 of a biocompatible material such as a woven Dacron. Along the length of the tubular body 3 there are self expanding Z stents 13 outside the tubular body stitched to the tubular body. At the proximal end 5 there is an internal self expanding Z stent 15 again stitched to the body. When the stent graft 1 is deployed into a vessel of a human or animal body such as an aorta this proximal Z stent 15 provides pressure against the wall of the aorta in the landing zone and as the Z stent 15 within the tubular body an essentially smooth outer surface is presented to the wall of the aorta to provide a degree of sealing.

At the proximal end 5 of the stent graft 1 there is also a proximally extending uncovered self expanding Z stent 7. This proximally extending Z stent 7 has barbs 9 extending from struts 11 of the stent 7. When the stent graft 1 is deployed into a vessel of a human or animal body such as an aorta the barbs 9 engage into the wall of the aorta and provide a purely mechanical immediate fixation of the stent graft into the vessel. There is a problem with migration, caused by pulsating blood flow for instance, which may cause the barbs to tear the wall of the aorta. An added long term biological fixation is therefore provided according to this invention.

The stent graft 1 includes a sheet of SIS material 60 in the form of a cuff or collar stitched onto the proximal end region 5 of the stent graft 1, in that portion which is intended to engage with the landing zone when deployed as discussed above, by means of wire stitches 62. The wire stitches 62 retain the SIS sheet material and have the added advantage that they provide apertures through the SIS and graft material which will permit cell growth through the material for enhanced biological fixation.

At least some 64 of the wire stitches 62 extend our from the surface of the SIS sheet 60 and provide a matrix of radially outward protruding portions when the stent graft is deployed and a stent associated with the graft is providing radially outward pressure. These protruding portions may create a localised pressure (greater than 30 mmHg-to overcome capillary pressure) against the aorta wall initiating cell necrosis. After cell death the protruding portions will continue to impinge on the arterial wall leading to endothelial wall remodelling to accommodate the protruding portions. Each protruding portion may in time become completely encapsulated by tissue growth. In effect, the protruding portions introduced on the outside of the graft serve as scaffold for tissue (adventitia) to grow around each protruding portions creating a significant fixation and sealing mechanism.

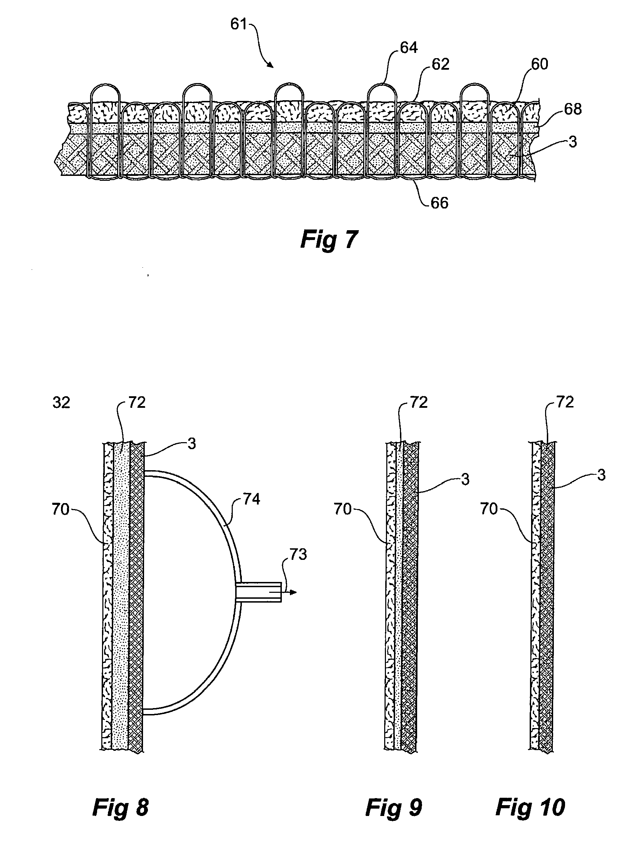

Figure 7 shows detail of the fastening of the sheet of SIS shown on Figure 6. In addition, however, in Figure 7 a SIS gel is used as a binder.

In this embodiment a SIS sheet is attached to a graft material by use of metal or synthetic biocompatible fibers or threads. SIS sheet can be stitched on to a synthetic graft so as to form a secure and permanent mechanical attachment. This technique can be enhanced by using a matrix of protruding stitches. As a further enhancement a SIS gel can then be used as a "binder" by following the steps described in the embodiment shown in Figures 8 to 10 to fill the pores created by the stitching process and further, the protruding fibers can form a mechanical scaffolding to hold the SIS gel in place. The resulting SIS stitched composite structure will encourage cell growth through the fiber loops employing all the benefits of attachment and fixation described above.

In Figure 7 the graft material 3 in the region 61 has a cuff or collar formed from a sheet of SIS 60 attached to it by stitching using stitches 62 which extend through both the SIS 60 and graft material 3 to a back stitch 66. An intermediate layer 68 of a SIS gel or digest can provide both a "glue" to connect the SIS sheet to the graft as well as promote further tissue-graft in-growth. The stitches can be formed from stainless steel wire, Nitinol wire or suture material. Some of the loops 64 of the stitches 62 extend beyond the SIS to assist with biological fixation by engaging the wall of a vessel into which they are deployed and encouraging tissue growth around the loops.

Figures 8 to 10 show a further embodiment of the invention to provide enhanced biological fixation.

In this embodiment a sheet of SIS 70 is laid over the graft material 3 in a selected region of the stent graft with an intermediate layer 72 of a SIS gel or digest. A vacuum 73 is applied by means of a vacuum hood 74 and the gel or digest 72 drawn into the graft material 3 to give the result shown in Figure 9 where the sheet of SIS 70 is adhered to the graft material 3 using the SIS gel 72 as an adhesive. The composite is then polymerized by heating in an oven at 37C for 20 minutes and the resultant product is freeze dried or vacuum pressed to provide a product as shown on Figure 10.

SIS sheet is a proven product capable of providing a collagen scaffolding enhanced with natural porcine growth factors and other proteins. This manufacturing technique utilizes the SIS sheet and attaches the sheet to the graft material in a secure fashion. The SIS gel is used as both a "glue" to connect the SIS sheet to the graft as well as promote further tissue-graft in-growth as with the embodiment discussed above. Hence one possible manufacturing process is as follows:
(a) apply the SIS digest and SIS sheet on to the outside of the graft and "vacuum-pull" the graft so that SIS gel can penetrate the graft.
(b) polymerize the composite graft in the oven at 37°C for 20 minutes.
(c) Freeze dry and /or vacuum press the composite graft.

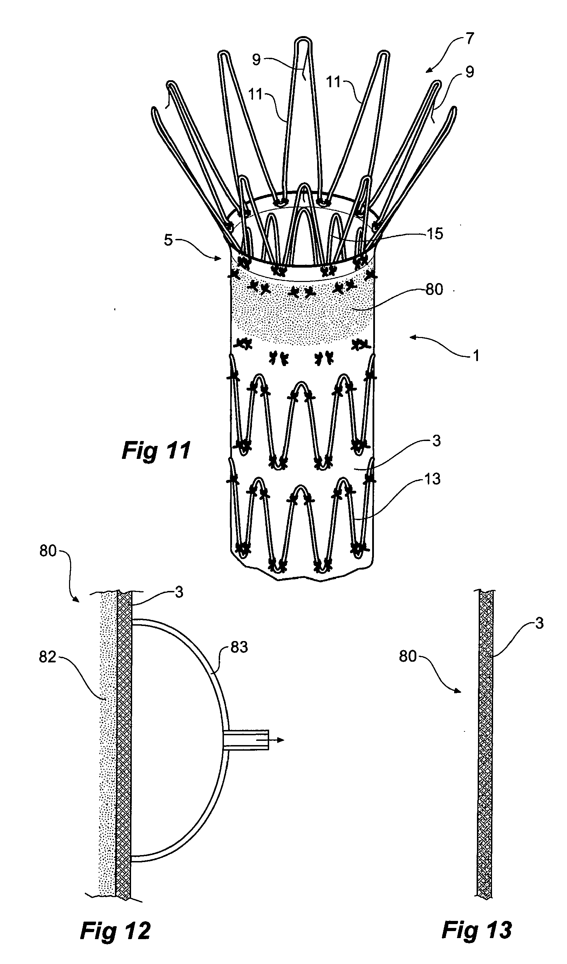

Figure 11 shows a perspective view of a portion of a stent graft incorporating the biological fixation arrangements according to a further embodiment of the present invention. In this embodiment the same reference numerals are used for corresponding components of the embodiment shown in Figure 1.

The stent graft 1 has a tubular body 3 of a biocompatible material such as a woven Dacron. Along the length of the tubular body 3 there are self expanding Z stents 13 outside the tubular body stitched to the tubular body. At the proximal end 5 there is an internal self expanding Z stent 15 again stitched to the body. When the stent graft 1 is deployed into a vessel of a human or animal body such as an aorta this proximal Z stent 15 provides pressure against the wall of the aorta in the landing zone and as the Z stent 15 within the tubular body an essentially smooth outer surface is presented to the wall of the aorta to provide a degree of sealing.

At the proximal end 5 of the stent graft 1 there is also a proximally extending uncovered self expanding Z stent 7. This proximally extending Z stent 7 has barbs 9 extending from struts 11 of the stent 7. When the stent graft 1 is deployed into a vessel of a human or animal body such as an aorta the barbs 9 engage into the wall of the aorta and provide a purely mechanical immediate fixation of the stent graft into the vessel. There is a problem with migration, caused by pulsating blood flow for instance, which may cause the barbs to tear the wall of the aorta. An added long term biological fixation is therefore provided according to this invention.

In the portion of the stent graft 1 just distal of the proximal end 5 there is a region 80 around the circumference of the stent graft which is intended to engage against the landing zone in use and which has a SIS gel or a digest of SIS impregnated through the graft material tube 3.

Figures 12 and 13 show this process in detail. A gel or digest of SIS 82 is applied to one surface of the graft material 3 in the region 80 and then a vacuum is applied by means of a vacuum hood 83 and the SIS gel or digest 82 is drawn into the graft material 3 to give the result shown in Figure 13. The natural porosity of the graft material can be altered by various weaving techniques to improve the amount of infusing into the graft of the SIS or by providing additional porosity in selected regions of the stent graft by drilling or ablating holes in the graft material using a laser or similar device. Such porosity may provide openings in the material of from 20 to 100 microns.

The cell wall tissue can then grow into the graft material via the scaffolding provided by the SIS. Fixation occurs as the synthetic fiber/fiber-bundles are surrounded by new tissue growth, such as the collagen based adventitia which forms a bio-mechanical attachment mechanism (between the graft and artery wall). Sealing will also be a by-product of the tissue growth through the SIS filled graft. In addition, it is likely that the endothelial cells will grow into the SIS on the inside of the graft wall forming a very smooth surface resulting in a non-thrombogenic graft property. Further, the resulting tissue in-growth will be biological active "alive" resulting in natural resistance to infection, as opposed to a purely synthetic graft.

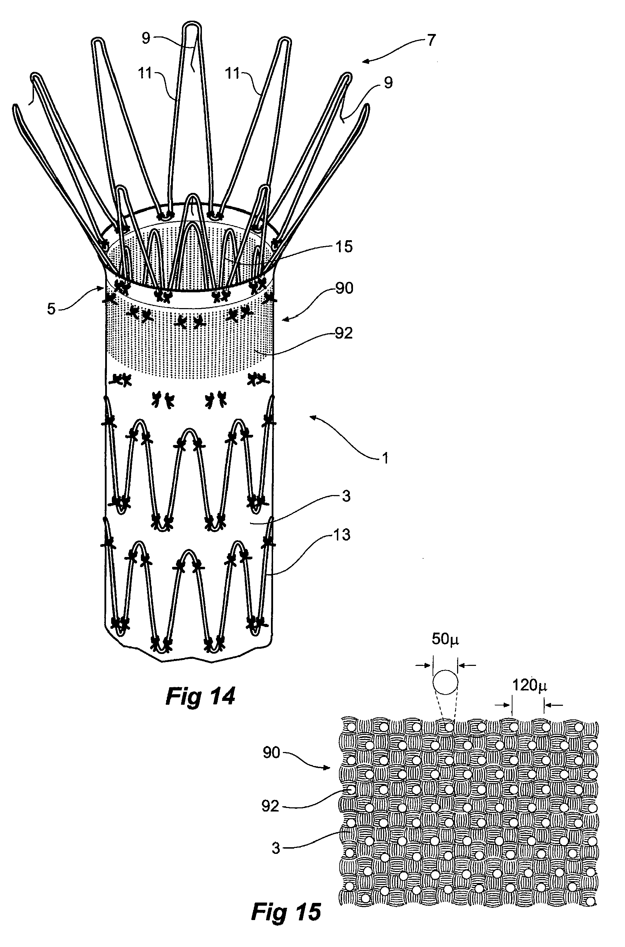

Figure 14 shows a perspective view of a portion of a stent graft incorporating the biological fixation arrangements according to an alternative embodiment ofthe present invention. In this embodiment the same reference numerals are used for corresponding components of the embodiment shown in Figure 1.
The stent graft 1 has a tubular body 3 of a biocompatible material such as a woven Dacron. Along the length of the tubular body 3there are self expanding Z stents 13 outside the tubular body stitched to the tubular body. At the proximal end 5 there is an internal self expanding Z stent 15 again stitched to the body. When the stent graft 1 is deployed into a vessel of a human or animal body such as an aorta this proximal Z stent 15 provides pressure against the wall of the aorta in the landing zone and with the Z stent 15 within the tubular body an essentially smooth outer surface is presented to the wall of the aorta to provide a degree of sealing.

At the proximal end 5 ofthe stent graft 1 there is also a proximally extending uncovered self expanding Z stent 7. This proximally extending Z stent 7 has barbs 9 extending from struts 11 of the stent 7. When the stent graft 1 is deployed into a vessel of a human or animal body such as an aorta the barbs 9 engage into the wall of the aorta and provide a purely mechanical immediate fixation of the stent graft into the vessel. There is a problem with migration with pulsating blood flow, for instance, which may cause the barbs to tear the wall of the aorta. An added long term biological fixation is therefore provided according to this invention.
In the region of the stent graft just distal of the proximal end 5 there is a region 90 around the circumference of the stent graft which has a selected porosity produced by a plurality of apertures 92 through the graft material tube 3.

It is intended to have the region 90 coinciding with the intended landing zone portion of the stent graft so that the existing low porosity of the graft material is maintained in the mid-graft region where the graft is open to the aneurysed region. Figure 15 shows an enlarged view of a portion of the graft material 3 of the stent graft 1. The material 3 in the region 90 has a plurality of apertures 92 ablated or drilled into it to provide the selected porosity. In this embodiment the apertures have a diameter of 50 microns and a spacing of 120 microns. As discussed earlier the apertures may have a diameter in the range of from 20 microns to 120 microns and a spacing of from 20 to 250 microns.

It could be advantageous to have a graft that is more porous at both the proximal and distal ends while retaining low porosity in the midgraft region. The ends of the graft come in contact with the arterial wall and are important for fixation and sealing.

Therefore, the more porous design in the specified regions could facilitate a bio-seal via cellular ingrowth and even edothelization on the internal surface of the graft wall. Tissue ingrowth through the laser drilled pores would also provide a bio-mechanical attachment resulting in significant long term fixation.

In a preferred embodiment of the invention the apertures can be produced by the ablation of graft material via a photochemical laser drilling process. The nature of this laser is to break chemical bonds layer-by-layer, thus vaporizing the polymer with minimal heating. Thus minimal damage is done to the surrounding graft material.

It is preferable to use some localized heating during the ablation process to provide a degree of fusing of fibre ends to enable the graft material to retain desired mechanical properties.

Throughout this specification various indications have been given as to the scope of this invention but the invention is not limited to any one of these but may reside in two or more of these combined together. The examples are given for illustration only and not for limitation.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.
The disclosure of EP 05757101.0 (published as WO 2005/112821), from which this application is divided, is incorporated herein by reference in its entirety.
Further embodiments are provided as follows:
The engagement portions may be formed onto the tubular body by stitching, adhesion of threading a further material or extra portions of the same material of the graft into the material of the tubular body.
The engagement portions may be resilient so that they can be compressed into a introducer device for endoluminal deployment.

The biologically active material may be associated with the synthetic biocompatible graft material by attaching a sheet thereof to the graft material by stitches of a metal or synthetic biocompatible fibre or thread.

At least some of the stitches of the metal or synthetic biocompatible fibre or thread may extend radially outward from the graft material.

The biologically active material may be associated with the synthetic biocompatible graft material by the use of a binder of a biologically active material gel or digest.

## Claims

1. A stent graft (1) comprising a substantially tubular body (3) of a biocompatible graft material and having a proximal end and a distal end, at least a portion along the length of the proximal end of the tubular body having a selected porosity to allow cell in-growth.

2. A stent graft as claimed in claim 1, wherein the porosity is provided by modification of the weave pattern of the biocompatible fibres which make up the graft material.

3. A stent graft as claimed in claim 1 or 2, wherein at least the proximal end (5) comprises a region intended in use to engage a landing zone in a vessel in the body, the region comprising a mechanical treatment to enhance biological fixation to the landing zone.

4. A stent graft as claimed in claim 1, 2 or 3, wherein the mechanical treatment comprises impregnation with a biologically active material.

5. A stent graft as claimed in claim 4, wherein the biologically active material is SIS gel (72) or digest (82).

6. A stent graft as claimed in any preceding claim, including apertures between the warp or weft fibres.

7. A stent graft as claimed in claim 1, wherein the porosity is provided by an array of apertures in the biocompatible graft material.

8. A stent graft as claimed in claim 6 or 7, wherein the apertures have a diameter in the range of from 20 microns to 120 microns and a spacing of from 20 to 250 microns.

9. A stent graft as claimed in claim 6 or 7, wherein the apertures have opening dimensions in the region of from 20 microns to 100 microns.
